# FASCICULE DE BREVET EUROPEEN

(11) **EP 0 774 963 B1**
(45) Date de publication et mention de la délivrance du brevet: **02.11.2000**
(21) Numéro de dépôt: 95925976.3
(22) Date de dépôt: 07.08.1995
(51) Int. Cl.: A61K 31/28

(54) **PREPARATION PHARMACEUTIQUEMENT STABLE D'OXALIPLATINE**
STABILES ARZNEIMITTEL ENTHALTEND OXALIPLATIN
PHARMACEUTICALLY STABLE OXALIPLATINUM PREPARATION

(30) Priorité: 08.08.1994 CH 246294
(43) Date de publication de la demande: 28.05.1997
(73) Titulaire: DEBIOPHARM S.A., 1000 Lausanne 9 (CH)
(72) Inventeur: IBRAHIM, Houssam, CH-1255 Veyrier (CH); MAUVERNAY, Rolland-Yves, CH-1003 Lausanne (CH)
(74) Mandataire: Micheli & Cie
(86) Numéro de dépôt international: IB9500614
(87) Numéro de publication internationale: WO9604904

(56) Documents cités:
- EP-A- 0 486 998
- WO-A-94/12193

## Description

La présente invention concerne une préparation pharmaceutiquement stable d'oxaliplatine pour administration par voie parentérale.

L'oxaliplatine (Dénomination Commune Internationale) est un isomère optique préparé en 1978 par Y. Kidani parmi un mélange de dérivés du diaminocyclohexane (dachplatine), à savoir le complexe cis-oxalato du platine II du trans-1-1,2-diaminocyclohexane ou selon "Who Drug Information", vol. 1, no 4, 1987, le (oxalato(2-)0,0') platine de la (1R,2R)-1,2-cyclohexane-diamine-N,N'. Ce composé complexe du platine est connu pour présenter une activité thérapeutique comparable, voire supérieure, à celle des autres composés complexes connus du platine, tel que le cisplatine par exemple.

Comme ce dernier, l'oxaliplatine est un agent antinéoplasique cytostatique utilisable dans le traitement thérapeutique de divers types de cancers, et plus particulièrement ceux du colon, des ovaires, des voies respiratoires supérieures, et les cancers épidermoïdes, ainsi que des tumeurs à cellules germinales (testicules, médiastin, glande pinéale,etc..). En plus des exemples mentionnés ci-dessus de l'utilisation de l'oxaliplatine, on peut en outre citer les cancers du colon résistant aux pyrimidines, cancers du poumon non à petites cellules, lymphomes non Hodgkiniens, cancers du sein, cancers des voies aérodigestives supérieures, mélanomes malins, hépatocarcinomes, cancers urothéliaux, cancers de la prostate, etc.. et de manière plus éparse d'autres types de tumeurs solides.

Actuellement, au stade des essais pré-cliniques et cliniques, l'oxaliplatine est présentée de préférence sous forme d'un lyophilisat pour perfusion en flacon, dont la préparation injectable est reconstituée avant administration avec de l'eau pour préparations injectables (ppi) ou du glucose à 5% isotonique, puis dilué dans un soluté glucose à 5%, pour pouvoir ensuite être administré par voie intraveineuse en perfusion.

Or, une telle présentation implique un procédé de fabrication (lyophilisation) qui est relativement complexe et coûteux, ainsi qu'une opération de reconstitution au moment de l'usage qui est délicate et nécessite une attention particulière. De plus, il s'est avéré qu'en pratique cette technique pouvait présenter des risques d'une mauvaise manipulation lors de la reconstitution extemporanée de la solution; en effet, il est courant d'utiliser pour reconstituer des préparations pharmaceutiques injectables à partir de lyophilisats, ou pour diluer des préparations liquides, une solution de NaCl 0,9%; or, l'utilisation par erreur d'une telle solution dans le cas de la forme lyophilisée de l'oxaliplatine serait très dommageable pour ce principe actif qui forme un précipité (dérivé dichlorodach-platine) avec le NaCl et conduit à la dégradation rapide dudit produit.

Ainsi, pour éviter tout risque de manipulation erronnée et fournir au médecin ou à l'infirmière une préparation d'oxaliplatine qui soit utilisable sans nécessiter les manipulations précitées, on a cherché à obtenir une solution injectable d'oxaliplatine prête à l'emploi, et qui reste en outre pharmaceutiquement stable avant utilisation pendant une durée acceptable selon les standards en vigueur, plus simple et moins coûteuse à fabriquer que le lyophilisat, tout en présentant une pureté chimique (pas d'isomérisation) et une activité thérapeutique équivalentes à celles du lyophilisat reconstitué. Ceci constitue le but de la présente invention.

Les présents inventeurs ont pu montrer que ce but pouvait être atteint, et ceci de manière tout à fait surprenante et inattendue, en utilisant comme forme galénique pour administration par voie parentérale une solution aqueuse de l'oxaliplatine, dont la concentration en principe actif et le pH se situent dans des plages de valeurs respectives bien déterminées, et qui soit libre de tout agent acide ou alcalin, de tampon ou d'autre additif. Il a notamment été constaté que des solutions aqueuses d'oxaliplatine ayant une concentration inférieure à environ 1mg/ml ne sont pas suffisamment stables.

En conséquence, l'objet de la présente invention consiste en une préparation pharmaceutiquement stable d'oxaliplatine pour administration par voie parentérale constituée par une solution d'oxaliplatine dans l'eau en une concentration de 1 à 5 mg/ml et dont le pH est de 4,5 à 6, la teneur en oxaliplatine dans la préparation représentant au moins 95% de la teneur initiale et la solution restant limpide, incolore et exempte de précipité après conservation pendant une durée pharmaceutiquement acceptable. Cette préparation est libre de tous autres constituants, et ne devrait en principe pas contenir plus de 2% environ d'impuretés.

De préférence, la concentration dans l'eau de l'oxaliplatine est d'environ 2 mg/ml, et le pH de la solution a une valeur moyenne d'environ 5,3.

La stabilité de la solution aqueuse de l'oxaliplatine a pu être également confirmée par la mesure du pouvoir rotatoire spécifique, dont la valeur se situe entre +74,5° et +78,0°.

Ainsi, il faut comprendre ici l'expression "stable pharmaceutiquement" également en ce qui concerne la stabilité du pouvoir rotatoire spécifique de l'oxaliplatine, c'est-à-dire la pureté optique de la solution (pas d'isomérisation). D'autre part, la durée "pharmaceutiquement acceptable" pendant laquelle la préparation selon l'invention doit rester stable doit être comprise ici comme correspondant aux standards généralement admis dans le domaine, c'est-à-dire par exemple pendant environ 3 à 5 ans à température ambiante ou à température du réfrigérateur.

La fabrication de la préparation selon l'invention peut être effectuée de préférence par dissolution de l'oxaliplatine dans de l'eau pour préparations injectables (ppi), éventuellement sous agitation contrôlée et préchauffage à environ 40°C, et suivie par une filtration de clarification et une ou plusieurs filtrations de stérilisation. Après remplissage et fermeture du conditionnement primaire choisi, la préparation peut encore être stérilisée par une étape d'autoclavage.

De préférence, la préparation selon l'invention est sous la forme d'une solution aqueuse d'oxaliplatine prête à l'emploi et contenue dans un récipient fermé hermétiquement.

Selon une forme particulière de l'invention, la préparation selon l'invention est présentée sous la forme d'une dose active unitaire injectable par perfusion de 50 ou de 100 mg d'oxaliplatine dans une quantité d'eau pour préparations injectables (ppi) choisie selon la concentration désirée.

Cette dose est avantageusement contenue dans un flacon en verre neutre pour usage pharmaceutique, fermé par un bouchon dont au moins la surface s'étendant à l'intérieur du flacon est inerte vis-à-vis de la solution aqueuse d'oxaliplatine, l'espace entre ladite solution et ledit bouchon étant éventuellement rempli d'un gaz inerte.

Le récipient fermé hermétiquement peut également être, par exemple, une poche souple pour perfusion, une ampoule ou encore un élément constitutif d'un dispositif de perfusion muni d'une micropompe d'injection.

La solution aqueuse d'oxaliplatine peut être administrée par voie intraveineuse à l'aide des instruments conventionnels, le cas échéant conjointement à d'autres agents thérapeutiquement actifs ou non, dans des conditions physico-chimiquement compatibles avec ce dérivé du platine, et conformément aux pratiques ayant cours en thérapie cancéreuse.

L'oxaliplatine peut être prescrit à des doses de 50 à 200 mg/m2 de surface corporelle, de préférence d'environ 100 à 130 mg/m2 par cycle, la durée de l'administration étant d'environ 2 à 5 heures, les cycles étant en général espacés de 3 à 5 semaines, et le traitement complet pouvant atteindre 6 à 10 cycles.

L'invention sera maintenant illustrée en référence aux exemples ci-après qui décrivent plus en détails la préparation injectable selon l'invention, sa fabrication et sa stabilité dans le temps.

### Exemple 1 : Préparation de la solution aqueuse d'oxaliplatine

Dans un récipient thermostabilisable en verre ou en acier inoxydable, on introduit environ le 80% de la quantité d'eau ppi désirée, dont la température est amenée à 40°C ± 5° sous agitation (800-1200 tpm).

La quantité d'oxaliplatine nécessaire pour obtenir une concentration par exemple de 2 mg/ml est pesée séparément, puis ajoutée dans l'eau préchauffée. Le récipient de pesage est rincé trois fois avec de l'eau ppi, laquelle est également ajoutée dans le mélange principal. Celui-ci est encore agité à la température précitée pendant 30 min ±5, éventuellement plus longtemps si nécessaire, c'est-à-dire jusqu'à complète dissolution de l'oxaliplatine. Selon une variante, l'eau utilisée peut être soumise à un barbotage d'azote pour diminuer sa teneur en oxygène.

La solution est ensuite ajustée à son volume ou son poids souhaité par addition d'eau ppi, puis homogénéisée pendant encore 10 min ±2 (800-1200 tpm), et enfin refroidie jusqu'a environ 30°C, toujours sous agitation. On recueille à ce stade des échantillons de la solution pour effectuer les tests et contrôles usuels, et on soumet la solution à une filtration aseptique de clarification de manière connue en soi, et on conserve la solution entre 15 et 30°C avant conditionnement.

De préférence, on utilise comme oxaliplatine de départ un produit apyrogène, de qualité pharmaceutique et optiquement pur (> 99,5%), par exemple tel qu'obtenu par le procédé breveté de Tanaka KK.

### Exemple 2 : Conditionnement

La solution aqueuse d'oxaliplatine, par exemple à 2 mg/ml, est ensuite conditionnée de manière aseptique et de préférence sous atmosphère inerte, par exemple d'azote, dans des flacons stérilisés en verre de 50 ml et dépyrogénéisés.

Pour obtenir une meilleure stabilité de la solution aqueuse d'oxaliplatine, on utilise de préférence du verre neutre de type I.

Comme bouchon, on peut utiliser par exemple des bouchons en Téflon ou en un élastomère à base de butyles halogénés, éventuellement pourvus d'un revêtement approprié, notamment en polymère fluoré (par exemple du type "Omniflex" de Helvoet Pharma), de telle sorte qu'au moins la surface s'étendant à l'intérieur du flacon soit inerte, vis-à-vis de la solution aqueuse d'oxaliplatine.

L'espace entre le bouchon et la solution aqueuse d'oxaliplatine peut éventuellement être rempli d'un gaz inerte, par exemple de l'azote.

### Example 3 : Tests de stabilité

Des tests de stabilité dans le temps ont été effectués sur des solutions aqueuses d'oxaliplatine obtenues comme décrit précédemment et conservées dans des conditions de conditionnement différentes, plus particulièrement avec deux types de bouchons différents, à savoir :
- Bouchon A :: "Omniflex"
- Bouchon A(N) :: "Omniflex" (avec espace rempli d'N₂)
- Bouchon B :: "Grey Butyl" ( idem )

Les tests ont été effectués sur 13 semaines et à plusieurs températures différentes, à savoir 5°C ± 3 (température du réfrigérateur), 27,5°C ±2,5 (température ambiante), 40° (à 75% d'humidité relative) et 50°C pour créer une "accélération artificielle" du phénomène de dégradation dans le temps de stockage; de plus, le test à 27,5°C a été répété en présence d'une source lumineuse importante (1100 lux).

Une des techniques employées est celle couramment pratiquée dans le domaine de l'art, à savoir par chromatographie liquide à haute performance (CLHP), par exemple selon la méthode décrite dans le J. of the Parenteral Drug Assoc., p. 108-109, 1979, afin notamment d'obtenir par analyse des pics chromatographiques la teneur et le % des impuretés, dont la principale identifiée est dans le cas présent l'acide oxalique. De plus, pour chaque essai, le pH, la couleur et l'opalescence de la solution ont été également mesurées par une méthode classique décrite dans la pharmacopée.

Les résultats obtenus, qui sont résumés sur le Tableau ci-après, démontrent que dans toutes les conditions testées la stabilité de la solution aqueuse d'oxaliplatine selon l'invention peut être considérée comme pharmaceutiquement acceptable, les % respectifs d'oxaliplatine et d'impuretés retrouvés, même après plus de 3 mois de conservation à 50°C, étant à des niveaux encore bien inférieurs à ceux requis, et le pH restant stable. De plus, toutes les solutions sont restées limpides, incolores et exemptes de particules solides visiblement décelables. Enfin, il a également été démontré que les solutions restaient en outre optiquement pures (pas d'isomérisation), le pouvoir rotatoire de l'oxaliplatine étant mesuré entre environ +75,7° et environ +76,2°, soit largement à l'intérieur des normes admises (+74,5 °à + 78,0°)

Une autre série de mesures à température ambiante et à 40°C a également confirmé la stabilité de la solution aqueuse d'oxaliplatine sur une période de plus de 10 mois.

**TABLEAU**

| Réf. du test (bouchon) | Cond. de stockage (°C) | Oxaliplatine retrouvé (% quant. initiale) | Impuretés (%) | pH |
|---|---|---|---|---|
| A | 5±3 | 101,0 | 0,18 | 5,35 |
| A(N) | 5±3 | 101,0 | 0,28 | 5,35 |
| B | 5±3 | 100,0 | 0,28 | 5,34 |
| A | 27,5±2,5 | 100,0 | 0,29 | 5,37 |
| A(N) | 27,5±2,5 | 100,0 | 0,31 | 5,33 |
| B | 27,5±2,5 | 100,5 | 0,37 | 5,36 |
| A | 27,5/1100 lux | 100,5 | 0,34 | 5,34 |
| A(N) | 27,5/1100 lux | 99,5 | 0,42 | 5,29 |
| B | 27,5/1100 lux | 100,0 | 0,40 | 5,37 |
| A | 40 (75% H.R.) | 100,0 | 0,35 | 5,46 |
| A(N) | 40 (75% H.R.) | 100,5 | 0,35 | 5,50 |
| B | 40 (75% H.R.) | 99,5 | 0,63 | 5,47 |
| A | 50 | 99,5 | 0,49 | 5,57 |
| A(N) | 50 | 99,0 | 0,54 | 5,65 |
| B | 50 | 99,0 | 1,16 | 5,59 |

## Revendications

1. Préparation pharmaceutiquement stable d'oxaliplatine pour administration par voie parentérale constituée par une solution d'oxaliplatine dans l'eau en une concentration de 1 à 5 mg /ml et dont le pH est de 4,5 à 6, la teneur en oxaliplatine dans la préparation représentant au moins 95% de la teneur initiale et la solution restant limpide, incolore et exempte de précipité après conserva tion pendant une durée pharmaceutiquement acceptable.

2. Préparation selon la revendication 1 dans laquelle la concentration en oxaliplatine est d'environ 2 mg/ml d'eau et le pH de la solution a une valeur moyenne d'environ 5,3.

3. Préparation selon la revendication 1 ou la revendication 2 dans laquelle la solution d'oxaliplatine a un pouvoir rotatoire spécifique de +74,5° à +78,0°.

4. Préparation selon l'une des revendications 1 à 3, sous la forme d'une solution aqueuse d'oxaliplatine prête à l'emploi et contenue dans un récipient fermé hermétiquement.

5. Préparation selon la revendication 4, caractérisée par le fait que ledit récipient contient une dose active unitaire et injectable par perfusion de 50 ou de 100 mg d'oxaliplatine.

6. Préparation selon la revendication 4 ou la revendication 5, caractérisée par le fait que ledit récipient est un flacon en verre pour usage pharmaceutique et fermé par un bouchon dont au moins la surface s'étendant à l'intérieur du flacon est inerte vis-à-vis de ladite solution.

7. Préparation selon la revendication 6, caractérisée par le fait que l'espace entre ladite solution et ledit bouchon est rempli par un gaz inerte.

8. Préparation selon la revendication 4 ou la revendication 5, caractérisée par le fait que ledit récipient est une poche souple pour perfusion ou une ampoule.

9. Préparation selon la revendication 4 ou la revendication 5, caractérisée par le fait que ledit récipient est un élément constitutif d'un dispositif de perfusion muni d'une micropompe d'injection.

## Claims

1. A pharmaceutically stable preparation of oxaliplatinum for a parenteral administration, provided as an oxaliplatinum solution in water at a concentration of 1 to 5 mg / ml and having a pH of 4.5 to 6, the oxaliplatinum content in the preparation amounting to at least 95 % of the initial content and the solution remaining clear, colourless and devoid of precipitate after a storage of a pharmaceutically acceptable duration.

2. A preparation according to claim 1, in which the oxaliplatinum concentration is of about 2 mg / ml of water and the pH of the solution has an average value of about 5.3.

3. A preparation according to claim 1 or claim 2, in which the oxaliplatinum solution has a specific optical rotation from + 74.5 ° to + 78.0 °.

4. A preparation according to one of claims 1 to 3, in the form of an aqueous solution of oxaliplatinum which is ready for use and which is contained in a hermetically closed container.

5. A preparation according to claim 4, characterised in that said container contains a unitary active dose injectable by infusion, of 50 or of 100 mg of oxaliplatinum.

6. A preparation according to claim 4 or claim 5, characterised in that said container is a glass vial for pharmaceutical use and is closed by a stopper of which at least the surface extending inside said vial is inert vis-à-vis said solution.

7. A preparation according to claim 6, characterised in that the space between said solution and said stopper is filled with an inert gas.

8. A preparation according to claim 4 or claim 5, characterised in that said container is a flexible pouch for infusion or an ampoule.

9. A preparation according to claim 4 or claim 5, characterised in that said container is a component element of an infusion device provided with an injection micropump.

## Patentansprüche

1. Pharmazeutisch stabile Zubereitung von Oxaliplatin für parenterale Verabreichung, bestehend aus einer Lösung von Oxaliplatin in Wasser in einer Konzentration von 1 bis 5 mg/ml, deren pH-Wert 4,5 bis 6 ist, wobei der Gehalt an Oxaliplatin in der Zubereitung zumindest 95 % des anfänglichen Gehalts darstellt und die Lösung nach Aufbewahrung über eine pharmazeutisch annehmbare Zeitdauer hinweg klar, farblos und frei von Niederschlag bleibt.

2. Zubereitung nach Anspruch 1, in der die Konzentration an Oxaliplatin etwa 2 mg/ml Wasser und der pH-Wert der Lösung im Mittel etwa 5,3 beträgt.

3. Zubereitung nach Anspruch 1 oder Anspruch 2, worin die Lösung von Oxaliplatin ein spezifisches Drehvermögen von 74,5° bis 78,0° besitzt.

4. Zubereitung nach einem der Ansprüche 1 bis 3 in Gestalt einer gebrauchsfertigen, in einem dicht verschlossenen Behältnis enthaltenen wässrigen Lösung von Oxaliplatin.

5. Zubereitung nach Anspruch 4, dadurch gekennzeichnet, dass das benannte Behältnis eine durch Infusion einspritzbare, aktive Einheitsdosis von 50 oder 100 mg Oxaliplatin enthält.

6. Zubereitung nach Anspruch 4 oder Anspruch 5, dadurch gekennzeichnet, das das benannte Behältnis ein Glasflakon zum pharmazeutischen Gebrauch ist und durch einen Stopfen verschlossen wird, von dem zumindest die sich ins Innere des Flakons erstreckende Seite gegenüber der benannten Lösung inert ist.

7. Zubereitung nach Anspruch 6, dadurch gekennzeichnet, dass der Raum zwischen der benannten Lösung und dem benannten Stopfen mit einem Inertgas gefüllt ist.

8. Zubereitung nach Anspruch 4 oder Anspruch 5, dadurch gekennzeichnet, dass das benannte Behältnis ein weicher Beutel für die Infusion oder eine Ampulle ist.

9. Zubereitung nach Anspruch 4 oder Anspruch 5, dadurch gekennzeichnet, dass das benannte Behältnis ein integrierender Bestandteil einer mit einer Mikroeinspritzpumpe ausgerüsteten Infusionsvorrichtung ist.
